# EUROPÄISCHE PATENTSCHRIFT

(11) **EP 1 006 932 B1**
(45) Veröffentlichungstag und Bekanntmachung des Hinweises auf die Patenterteilung: **23.04.2003**
(21) Anmeldenummer: 97911121.8
(22) Anmeldetag: 30.09.1997
(51) Int. Cl.: A61C 13/00, A61B 5/00

(54) **VORRICHTUNG ZUR KONFOKALEN OBERFLÄCHENVERMESSUNG**
CONFOCAL SURFACE-MEASURING DEVICE
DISPOSITIF DE MESURE CONFOCALE DE SURFACES

(30) Priorität: 01.10.1996 DE 19640495
(43) Veröffentlichungstag der Anmeldung: 14.06.2000
(73) Patentinhaber: Leica Microsystems Heidelberg GmbH, 69120 Heidelberg (DE)
(72) Erfinder: ENGELHARDT, Johann, D-76669 Bad Schönborn (DE); ZAPF, Thomas, D-67346 Speyer (DE)
(74) Vertreter: Stamer, Harald
(86) Internationale Anmeldenummer: DE9702240
(87) Internationale Veröffentlichungsnummer: WO98014132

(56) Entgegenhaltungen:
- WO-A-91/03988
- WO-A-95/25460
- US-A- 4 638 800

## Beschreibung

Die Erfindung betrifft eine Vorrichtung zur konfokalen Oberflächenvermessung in Körperhöhlen, insbesondere zur Vermessung des Oberflächenprofils von Zähnen in der Mundhöhle, mit einer in die Körperhöhle einführbaren Sonde, einer die Sonde speisenden Lichtquelle, einem Lichtsignale aufnehmenden Detektor und einem die detektierten Signale digitalisierenden und zu einer dreidimensionalen Darstellung weiterverarbeitenden Prozessor.

Der Erfindung liegt ein Verfahren zur Vermessung von Oberflächen jedweder Art und jedweder Kontur zugrunde, wobei aus der Praxis unterschiedliche Verfahren zur Oberflächenvermessung bekannt sind.

So läßt sich beispielsweise mittels eines Lichtschnittsensors eine Lichtlinie auf das Objekt projizieren und mit einer CCD-Kamera unter einem Winkel beobachten. Die geometrische Verformung der Lichtlinie wird dabei gemessen. Aus dieser Verformung werden die Höhendifferenzen auf dem Objekt berechnet. Durch Verschiebung des Objekts unter dem Sensor - senkrecht zur Lichtlinie - und durch wiederholte Messung eines Profils läßt sich seriell die Oberflächenform vermessen beziehungsweise bestimmen.

Zwar handelt es sich bei dem Lichtschnittsensor um einen einfach aufgebauten und dabei robusten Sensor, jedoch führt die hier erforderliche Schrägbeleuchtung zu einer einseitigen Abschattung steiler Stellen. Dadurch entstehen Asymmetrien in der Abbildung beziehungsweise Ungenauigkeiten. Des weiteren werden durch Streuung von Licht aus verschiedenen Tiefen beispielsweise eines zumindest teiltransparenten Zahnmaterials die Messungen abermals ungenau beziehungsweise verfälscht.

Auch ist bereits eine Vorrichtung zur Vermessung des Oberflächenprofils von Zähnen in der Mundhöhle bekannt, wobei diese Vorrichtung aus den Hauptkomponenten Kamera, Bildschirm und Rechner besteht. Diese Vorrichtung ist unmittelbar einer Schleifeinheit zur Anfertigung eines Inlays vorgeschaltet (vgl. Dr. Klaus J. Wiedhahn in DENTAL MAGAZIN 1/95 "Cerec 2 - eine neue Epoche?").

Bei der bekannten Vorrichtung zur Vermessung des Oberflächenprofils von Zähnen ist die Kamera bzw. Sonde derart konzipiert, daß Infrarotlicht über ein oszillierendes Strichgitter geleitet wird, um dann auf der mit weißem TiO₂-Puder beschichteten Zahnoberfläche reflektiert in einem symmetrischen Strahlengang auf den in der Kamera befindlichen CCD-Sensor geleitet zu werden. Pro Aufnahmesequenz (0,2 s) werden vier Einzelaufnahmen unter differierenden Strichgitterwinkeln gemacht und die vier Einzelbilder werden zu einem dreidimensionalen Abbild des Zahnes umgerechnet. Der mit der Kamera gewonnene dreidimensionale "optische Abdruck" wird auf einem feinzeichnenden Farbmonitor als pseudoplastisches Bild dargestellt und die gewonnenen Bild- und Konstruktionsdaten werden im Bildverarbeitungs-Rechenwerk und den eingebauten Prozessoren verarbeitet und an die Schleifeinheit weitergeleitet.

Das hier in Rede steende bekannte Verfahren und die damit verbundene Hardware ist jedoch insoweit problematisch, als es stets erforderlich ist, die Zahnoberfläche mit einem Pulver bzw. Puder zu beschichten, um nämlich eine eindeutige Reflexion an der Zahnoberfläche zu gewährleisten. Des weiteren ist die Kamera mit dem dort verwendeten CCD-Sensor konstruktiv aufwendig.

Schließlich ist es aus der Praxis auch bereits bekannt, mittels konfokaler Mikroskopie Oberflächen zu scannen und daraus dreidimensionale Aufnahmen der Oberfläche zu generieren. Hierzu wird lediglich beispielhaft auf Johann Engelhardt und Werner Knebel in Physik in unserer Zeit, 24. Jahrg. 1993, Nr. 2 "Konfokale Laserscanning-Mikroskopie" und auf D.K. Hamilton und T. Wilson in Appl. Phys. B 27, 211-213, 1982 "Three-Dimensional Surface Measurement Using the Confocal Scanning Microscope" verwiesen. Hinsichtlich eines entsprechenden Systems - Leica TCS NT - wird auf den Leica-Prospekt "The Confokal System, Leica TCS NT", und zwar hinsichtlich der Anwendung im Dentalbereich insbesondere auf Seite 16, verwiesen. Eine solche Vorrichtung ist jedoch einerseits zu groß zur Applikation in der Mundhöhle eines Patienten und andererseits in konstruktiver Hinsicht zu aufwendig und somit zu teuer im Rahmen einer zahnärztlichen Anwendung.

Ungeachtet der voranstehend genannten Nachteile eignet sich die konfokale Mikroskopie zur Oberflächenvermessung von Zahnoberflächen ganz besonders, da nach diesem Verfahren lediglich diejenigen Strukturen abgebildet werden, die sich unmittelbar in der Brennebene des Mikroskopobjektivs befinden. Meßfehler aufgrund des teiltransparenten Zahnmaterials sind somit wirksam vermieden. Allerdings versagt die Methode der Reflektionsmessung mit dem herkömmlichen Konfokalmikroskop bei steilen Übergängen beziehungsweise Flanken, wenn deren Winkel größer als der Aperturwinkel des Objektivs ist, da nämlich dann der Rückreflex das Objektiv nicht mehr trifft und somit für die Auswertung verloren geht (vergleiche P.C. Cheng und R.G. Summers in Confocal Microscopy Handbook, Chapter 17).

Der vorliegenden Erfindung liegt nun die Aufgabe zugrunde, eine Vorrichtung zur konfokalen Oberflächenvermessung anzugeben, mit der eine dreidimensionale Abtastung von Oberflächen in Körperhöhlen wie z.B. der Oberfläche eines Zahnes in der Mundhöhle eines Patienten möglich ist. Dabei soll die in die Mundhöhle einzuführende Sonde hinreichend klein und einfach in der Konstruktion sein.

Die erfindungsgemäße Vorrichtung zur Oberflächenvermessung löst die voranstehende Aufgabe durch die Merkmale des Patentanspruches 1. Danach ist die hier in Rede stehende Vorrichtung zur konfokalen Oberflächenvermessung in Körperhöhlen, insbesondere zur Vermessung des Oberflächenprofils von Zähnen in der Mundhöhle, derart ausgestaltet, daß die Sonde im Sinne eines Rotationsscanners mit mindestens einer Umlenkeinrichtung ausgebildet ist, die den Beleuchtungsstrahl in Richtung der zu vermessenden Oberfläche lenkt, daß die Umlenkeinrichtung zum Vorschub des rotierenden Beleuchtungsstrahls in einer weiteren zu scannenden Achse verschiebbar ist und daß der Detektor eine Einrichtung zur sequentiellen oder simultanen Abtastung mehrerer Brennebenen sowohl hinsichtlich spekularer Reflexe als auch hinsichtlich schwachen Streulichts oder Fluoressenzlichts der jeweiligen Brennebene umfaßt.

Für die erfindungsgemäße Vorrichtung ist von ganz besonderer Bedeutung, daß hier das Prinzip der konfokalen Mikroskopie zugrundeliegt und daß eine sequentielle oder simultane Abtastung mehrerer Brennebenen sowohl hinsichtlich spekularer Reflexe als auch hinsichtlich schwachen Streulichts oder Fluoressenzlichts der jeweiligen Brennebene stattfindet. Bevor nun die ganz besonderen konstruktiven Ausgestaltungen der erfindungsgemäßen Vorrichtung erläutert werden, sei nachfolgend die grundsätzliche Funktionsweise in Bezug auf die konfokale Oberflächenvermessung und die Abtastung hinsichtlich spekularer Reflexe wie auch hinsichtlich schwachen Streulichts oder Fluoressenzlichts erörtert.

Danach handelt es sich hier um eine Vorrichtung zur Oberflächenvermessung mittels Konfokalmikroskopie im Reflexionsverfahren, insbesondere zur Vermessung des Oberflächenprofils von bearbeiteten beziehungsweise gebohrten Zähnen, welche durch eine konfokale Abbildung mit hoher Dynamik (relative Empfindlichkeit) zur Abbildung einerseits spekularer Reflexe und andererseits schwachen Streulichts oder Fluoreszenzlichts der jeweiligen Brennebene geprägt ist.

Hinsichtlich des hier zugrundeliegenden Verfahrens ist demnach erkannt worden, daß sich die Konfokalmikroskopie ganz besonders zur Oberflächenvermessung teiltransparenter Materialien eignet, da nämlich bei der Konfokalmikroskopie lediglich diejenigen Strukturen abgebildet werden, die sich unmittelbar in der jeweiligen Brennebene des Mikroskopobjektivs befinden. Des weiteren ist erkannt worden, daß sich der Nachteil herkömmlicher Konfokalmikroskopie im Reflexionsverfahren hinsichtlich der zuvor erörterten Aperturproblematik dadurch beheben läßt, daß man zu der üblichen Auswertung des Rückreflexes nunmehr auch eine Auswertung des Streulichts oder Fluoreszenzlichts der jeweiligen Brennebene vornimmt.

Zur Realisierung einer Auswertung des Streulichts beziehungsweise Fluoreszenzlichts kann die konfokale Abbildung mit hoher Dynamik, d.h. mit hoher relativer Empfindlichkeit erfolgen, so daß einerseits eine Abbildung stark reflektierter flacher Flächen und andererseits eine Darstellung des Streulichts oder Fluoreszenzlichts auch an steilen Flanken möglich ist. Folglich ist eine Abbildung auch dann möglich, wenn das an steilen Stellen reflektierte Licht am Objektiv vorbei reflektiert wird, so daß - im üblichen Reflexionsverfahren - keine Profilometrie betrieben werden kann. Letztendlich wird das Streulicht zur Auswertung stets dann hinzugezogen, wenn eine Abbildung mangels spekularer Reflexe nach herkömmlicher Konfokalmikroskopie nicht mehr möglich ist.

Wie bereits zuvor erwähnt, erfolgt die Digitalisierung der Detektionssignale mit hoher Auflösung, und zwar möglichst bei einer Dynamik wesentlich größer als 8 bit. Zur ganz besonders wirksamen Nutzung des schwachen Streulichts beziehungsweise Fluoreszenzlichts im Bereich steiler Stellen der Oberfläche kann die relative Empfindlichkeit beziehungsweise Dynamik der konfokalen Abbildung bei 16 bit liegen. Letztendlich läßt sich hierdurch ein großer Helligkeitsunterschied durch Streulichtauswertung im Bereich steiler Flanken erzeugen.

Zur Höhenauswertung beziehungsweise zur Erzeugung des Oberflächenprofils anhand des schwachen Streulichts ist ein Algorithmus vorgesehen, der die hohe Dynamik des Systems berücksichtigt beziehungsweise toleriert. Dieser Algorithmus berücksichtigt interpolierend nächste beziehungsweise unmittelbare Nachbarebenen, wobei höhere Intensitäten in einem lokalen Bereich relativ übergewichtet werden, um nämlich die Signaluntergrundabhängigkeiten zu reduzieren. Letztendlich ist ein geeigneter Algorithmus vorgesehen, der nach Detektion der Streulichtsignale sowie nach Digitalisierung mit hoher Auflösung eine adäquate Höhenauswertung der digitalisierten Signale vornimmt.

An dieser Stelle sei hervorgehoben, daß das Scannen der Oberfläche auch in Dunkelfeldanordnung erfolgen kann. Als Lichtquelle könnte entweder eine Punktlichtquelle oder eine Lichtquelle mit entsprechender Ausblendung vorgesehen sein.

Im Rahmen einer Anwendung in der Zahnheilkunde, insbesondere zur Herstellung passgenauer Inlays anstelle herkömmlicher Amalgamfüllungen, ist es von ganz besonderem Vorteil, wenn zunächst die Oberfläche des unbearbeiteten Zahnes abgescannt und die detektierten Werte - vorzugsweise digitalisiert und bereits zum Höhenprofil umgerechnet - gespeichert werden. In einem nächsten Schritt wird der Zahn bearbeitet beziehungsweise gebohrt. Danach erfolgt ein abermaliges Scannen des nunmehr bearbeiteten beziehungsweise gebohrten Zahnes sowie ebenfalls ein Abspeichern der das Oberflächenprofil des bearbeiteten Zahnes ergebenden Werte. Aus der sich ergebenden Differenz beider Oberflächenprofile beziehungsweise der die Oberflächenprofile aufspannenden Werte ergibt sich die Oberfläche beziehungsweise ergeben sich die genauen Maße des benötigten Inlays für eine optimale Okklusion des behandelten Zahnes.

Zum Erhalt einer besonders hohen Präzision bei der Bearbeitung des Inlays ist es von ganz besonderem Vorteil, wenn das zu fertigende Inlay nach einer ersten Bearbeitung gescannt wird und wenn die weitere Bearbeitung unter Hinzuziehung der anhand eines Soll/Ist-Vergleichs gewonnenen Korrekturwerte erfolgt. Insoweit ist eine Korrektur zur Verifizierung der Inlayform möglich, so daß bei Wiederholung dieses Vorgangs eine hohe Präzision der Fertigung des Inlays und somit eine optimale Okklusion möglich ist. Durch die voranstehende Maßnahme lassen sich obendrein geräte- beziehungsweise werkzeugbedingte Ungenauigkeiten, beispielsweise aufgrund von Abnutzungserscheinungen am Werkzeug berücksichtigen, so daß auch bei einer Dynamik in der Bearbeitungsstation eine optimale Anpassung des Inlays und somit eine optimale Okklusion möglich ist.

Des weiteren ist es möglich, daß - in einem weiteren Schritt - die im Zahn angelegte Bohrung mit einer plastischen Masse gefüllt wird, wobei durch Draufbeissen des Patienten die Berührungspunkte mit den gegenüberliegenden Zähnen in der plastischen Masse markiert werden. Das sich dabei ergebende Oberflächenprofil wird anschließend abgescannt, die erhaltenen Meßwerte betreffend das Oberflächenprofil werden gespeichert und bei der Berechnung der Oberfläche beziehungsweise der Maße des zu bearbeitenden Inlays berücksichtigt.

Für die erfindungsgemäße Vorrichtung ist es nun in konstruktiver Hinsicht von ganz besonderer Bedeutung, daß die Sonde im Sinne eines Rotationsscanners ausgebildet ist, daß nämlich der Beleuchtungsstrahl in rotierender Weise die zu vermessende Oberfläche bzw. den Zahn abscannt, wobei die hier zu scannende "Fokalebene" aufgrund der rotierenden Bewegung des Beleuchtungsstrahls in Form eines Zylindersegments ausgebildet ist. Entsprechend ist bei der weiteren Datenverarbeitung eine Transformation der Koordinaten der abgetasteten Zylindersegmente in eine echte Fokalebene, d.h. eine Geometriekorrektur, erforderlich, worauf später noch Bezug genommen wird.

Die im Sinne eines Rotationsscanners ausgebildete Sonde umfaßt eine Umlenkeinrichtung mit mindestens einer Refiexionsfläche, die den Beleuchtungsstrahl in Richtung der zu vermessenden Oberfläche lenkt. Die Umlenkeinrichtung bzw. die Reflexionsfläche ist zum Vorschub des durch Umlenkung rotierenden Beleuchtungsstrahls in einer weiteren zu scannenden Achse verschiebbar, so daß der rotierende Beleuchtungsstrahl bei gleichzeitiger linearer Verschiebung der Umlenkrichtung eine spiralförmige bzw. schraubenlinienförmige Bewegung ausführt.

Um nun im Verlaufe der Oberflächenabtastung das gesamte Oberflächenprofil mit unterschiedlichen Höhen, d.h. auf unterschiedlichen Brennebenen mit unterschiedlichen Brennpunkten abtasten zu können, ist des weiteren eine Einrichtung zur sequentiellen oder simultanen Abtastung mehrerer Brennebenen erforderlich, wobei diese Vorrichtung vorzugsweise dem Detektor zugeordnet ist. Wesentlich ist hierfür jedenfalls, daß das Objekt durch den Beleuchtungsstrahl über einen vorgebbaren Fokusbereich beleuchtet bzw. gescannt wird. Das nach der Wechselwirkung mit dem Objekt bzw. der Oberfläche des Objekts zurückkehrende Licht wird durch eine Sammeloptik im Bildbereich fokussiert, wo in mehreren Bildebenen im wesentlichen zentral liegende Anteile des dort fokussierten Lichts in Richtung der Detektionsmittel gelenkt werden. Ungeachtet einer konkreten Ausgestaltung der Einrichtung zur sequentiellen oder simultanen Abtastung mehrerer Brennebenen ist jedenfalls wesentlich, daß eine solche Abtastung - sequentiell oder simultan - sowohl hinsichtlich spekularer Reflexe als auch hinsichtlich schwachen Streulichts oder Fluoressenzlichts stattfindet.

Hinsichtlich einer konkreten Ausgestaltung der erfindungsgemäßen Vorrichtung, insbesondere hinsichtlich einer konkreten Ausgestaltung der Sonde und deren Innenleben unter dem Gesichtspunkt einer einfachen Konstruktion ist es von ganz besonderem Vorteil, wenn die Umlenkeinrichtung als Einfachspiegel ausgeführt ist. Dieser Einfachspiegel ist zur Umlenkung des Beleuchtungsstrahls unter einem entsprechenden Winkel zum Beleuchtungsstrahl drehbar bzw. rotierbar angeordnet. Ebenso könnte die Umlenkeinrichtung als Prisma oder als Polygon mit mehreren Facetten zur Bildung mehrerer Strahlführungen ausgebildet sein. Im Falle der Ausgestaltung als Polygon mit mehreren Facetten lassen sich durch entsprechende Anordnung kleinste lineare Vorschübe orthogonal zu der durch den rotierenden Beleuchtungsstrahl aufgespannten Ebene realisieren, worauf später noch Bezug genommen wird.

Im konkreten kann die Sonde ein zur Einführung in die Mundhöhle dienendes Gehäuse umfassen, wobei innerhalb des Gehäuses ein die Umlenkeinrichtung aufweisender drehbarer Rotor vorgesehen ist. Die Umlenkeinrichtung ist dabei im Bereich eines Beleuchtungs- und Detektionsfensters des Gehäuses angeordnet, so daß der zu dem zu scannenden Objekt hin gerichtete Beleuchtungsstrahl zumindest in einem durch das Beleuchtungs- und Detektionsfenster vorgegebenen Bereich ungehindert zu der abzutastenden Oberfläche gelangen kann. Der reflektierte Detektionsstrahl gelangt entsprechend wieder durch das Beleuchtungs- und Detektionsfenster über die Umlenkeinrichtung in die Sonde zurück bis hin zu einem später noch erläuterten Detektor.

Innerhalb des Rotors ist des weiteren eine den parallel zur Rotationsachse verlaufenden Beleuchtungsstrahl fokussierende Optik angeordnet. Diese Optik dreht gemeinsam mit dem Rotor, so daß eine rotationssymmetrische Ausgestaltung erforderlich ist, wobei die Optik und die Umlenkeinrichtung aufgrund der Anordnung in bzw. an dem Rotor gemeinsam drehen.

Im Rahmen einer besonders vorteilhaften und dabei einfachen Ausgestaltung ist der Rotor innerhalb des Gehäuses frei drehbar und linear verschiebbar geführt bzw. gelagert. Zur Drehbewegung und zum linearen Vorschub des Rotors ist ein besonderer Rotorantrieb vorgesehen, wobei für die Drehbewegung und für den linearen Vorschub des Rotors auch zwei unabhängige Antriebe verwendbar sind. Im Rahmen einer ganz besonders vorteilhaften Ausgestaltung sind die Drehbewegung und der lineare Vorschub des Rotors zwangsgekoppelt, nämlich dadurch, daß der Rotor mit einem Außengewinde in einem Innengewinde des Gehäuses drehbar geführt ist. Um nun in kleinsten Abständen die Oberflächen abtasten zu können, sind die Gewinde als Feingewinde mit geringer Steigung augeführt, wobei solche Feingewinde zu einem kleinstmöglichen linearen Vorschub jedenfalls dann ausreichen, wenn es sich bei der Umlenkeinrichtung um ein Polygon mit mehreren Facetten handelt, so daß das Zusammenspiel zwischen geringer Steigung und der geometrischen Anordnung der Facetten zu einem linearen Vorschub im Bereich von etwa 500 Micrometer pro Scanzeile führen kann.

Des weiteren ist es möglich, die Gewinde als Differentialgewinde mit sehr kleinem Vorschub auszugestalten, wonach sich ein Vorschub im Bereich von vorzugsweise 50 Micrometer pro Scannzeile realisieren läßt. Die Zwischenschaltung einer weiteren Einrichtung zur Unter- bzw. Übersetzung des linearen Vorschubs sind möglich.

Der zur Drehbewegung und/oder zum linearen Vorschub dienende Rotorantrieb ist in vorteilhafter Weise dem Gehäuse zugeordnet und wirkt unmittelbar zwischen dem Gehäuse und dem Rotor. So könnte dieser Antrieb bspw. bei fester Verbindung mit dem Gehäuse den Rotor umgreifen und im Falle einer Ausgestaltung des Rotors mit einem Außengewinde unmittelbar auf das Außengewinde im Sinne eines Spindelanatriebs zugreifen. Auch hier lassen sich weitere Antriebs- bzw. Kraftübertragungsvarianten realisieren.

Die zur Bereitstellung des Beleuchtungsstrahls dienende Lichtquelle könnte als Laserlichtquelle, insbesondere als Diodenlaser, ausgeführt sein. Dabei kann der Lichtquelle im Beleuchtungsstrahlengang ein Strahlteiler und eine Einrichtung zur Fokuskontrolle bzw. zur Brennweitenänderung des Beleuchtungsstrahls nachgeordnet sein. Sofem es sich bei der Lichtquelle um eine polyfokale Lichtquelle, d.h. um eine Lichtquelle mit unterschiedlichen Brennweiten, handelt, erübrigt sich die Vorkehrung der Fokuskontrolle, so daß sich dadurch die Vorrichtung abermals vereinfachen läßt. Entsprechend wäre dann der Lichtquelle im Beleuchtungsstrahlengang lediglich der Strahlteiler nachgeordnet und würde es sich bei dem Detektor entsprechend um einen polyfokalen Detektor handeln.

Gemäß voranstehender Beschreibung ist die Sonde im wesentlichen durch das Gehäuse - in räumlicher Hinsicht - definiert. Die Einspeisung des Lichts in die Sonde und die Ableitung der durch Wechselwirkung an der Oberfläche des abzutastenden Objekts entstandenen Signale aus der Sonde erfolgt in vorteilhafter Weise über eine Lichtleitfaser.

Ebenso wäre es jedoch denkbar, daß weitere gemäß voranstehender Beschreibung außerhalb des Gehäuses angeordnete Funktionseinheiten in das Gehäuse integriert bzw. innerhalb des Gehäuses angeordnet sind. So könnte die Lichtquelle und/oder der Strahlteiler und/oder - falls erforderlich - die Fokuskontrolle und/oder der Detektor und/oder der Prozessor im Rahmen einer Miniaturisierung sämtlicher Funktionseinheiten innerhalb des Gehäuses angeordnet sein. Entsprechend handelt es sich dann um eine kompakte Vorrichtung, die lediglich mit einer entsprechenden Energieversorgung zu koppeln ist. Im Rahmen einer einfachen Ausgestaltung der Sonde ist diese jedoch auf die wesentlichen Funktionseinheiten reduziert, so daß die Sonde über eine Lichtleitfaser mit den weiterverarbeitenden Einheiten und auch mit der Lichtquelle verbunden ist.

Schließlich sei darauf hingewiesen, daß der Prozessor gleich mehrere Aufgaben übemimmt, nämlich zur Steuerung, Transformation bzw. Geometriekorrektur und Digitalisierung der Signale, zur Berechnung des dreidimensionalen Oberflächenprofils sowie zur Speicherung der Daten dient.

Es gibt nun verschiedene Möglichkeiten, die Lehre der vorliegenden Erfindung in vorteilhafter Weise auszugestalten und weiterzubilden. Dazu ist einerseits auf die dem Patentanspruch 1 nachgeordneten Ansprüche, andererseits auf die nachfolgende Erläuterung dreier Ausführungsbeispiele der Erfindung anhand der Zeichnung zu verweisen. In Verbindung mit der Erläuterung der bevorzugten Ausführungsbeispiele der Erfindung werden auch im allgemeinen bevorzugte Ausgestaltungen und Weiterbildungen der Lehre erläutert. In der Zeichnung zeigt
- Fig. 1: in einer schematischen Darstellung ein erstes Ausführungsbeispiel einer erfindungsgemäßen Vorrichtung,
- Fig. 2: in schematischer Darstellung ein zweites Ausführungsbeispiel einer erfindungsgemäßen Vorrichtung,
- Fig. 3: in schematischer Darstellung ein drittes Ausführungsbeispiel einer erfindungsgemäßen Vorrichtung,
- Fig. 4: in einer schematischen Darstellung die Abtastung eines Objekts nach Umlenkung des Beleuchtungsstrahls an einem Polygon, wobei das Zusammenspiel von rotierender Bewegung und linearem Vorschub des Beleuchtungsstrahls zu einer Abtastung in Zylindersegmenten führt und
- Fig. 5: in einer schematischen Darstellung das Ergebnis der Abtastung nach Transformation bzw. Geometriekorrektur.

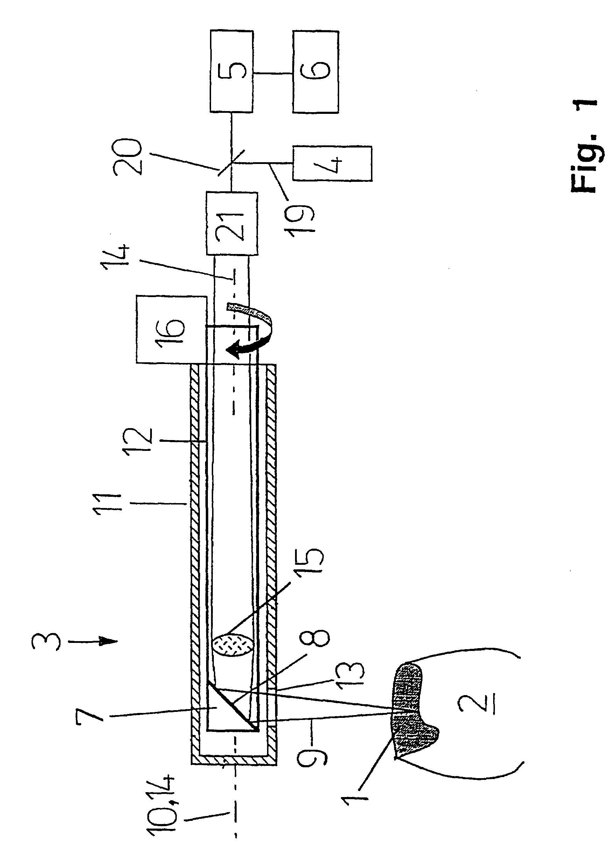

Die Figuren 1 bis 3 zeigen drei unterschiedliche Ausführungsbeispiele einer erfindungsgemäßen Vorrichtung zur konfokalen Oberflächenvermessung des Oberflächenprofils 1 von Zähnen 2 in einer hier nicht dargestellten Mundhöhle.

Die Vorrichtung umfaßt eine in die Körperhöhle einführbare Sonde 3, eine die Sonde 3 speisende Lichtquelie 4, einen Lichtsignale aufnehmenden Detektor 5 und einen die detektierten Signale digitalisierenden und zu einer dreidimensionalen Darstellung weiterverarbeitenden Prozessor 6.

Erfindungsgemäß ist die Sonde 3 im Sinne eines Rotationsscanners ausgebildet, wobei die Sonde 3 eine Umlenkeinrichtung 7 mit einer Reflexionsfläche 8 umfaßt. Die Umlenkeinrichtung 7 lenkt den Beleuchtungsstrahl 9 in Richtung der zu vermessenden Oberfläche 1, wobei die Umlenkeinrichtung 7 zum Vorschub des rotierenden Beleuchtungsstrahls 9 in einer weiteren zu scannenden Achse 10 verschiebbar ist.

Der Detektor 5 weist eine in den Figuren nicht gezeigte Einrichtung zur sequentiellen oder simultanen Abtastung mehrerer Brennebenen sowohl hinsichtlich spekularer Reflexe als auch hinsichtlich schwachen Streulichts oder Fluoreszenzlichts der jeweiligen Brennebene auf, so daß sich die gesamte Probe bzw. der Zahn 2 dreidimensional abtasten läßt.

Gemäß der Darstellung in den Figuren 1, 2 und 3 ist die Umlenkeinrichtung 7 als Einfachspiegel mit einer einzigen Reflexionsfläche 8 ausgeführt. Hinsichtlich weiterer möglicher Ausgestaltungen wird zur Vermeidung von Wiederholungen auf die allgemeine Beschreibung verwiesen.

Die in den Figuren 1 bis 3 gezeigten Ausführungsbeispiele haben gemeinsam, daß die Sonde 3 ein Gehäuse 11 mit einem die Umlenkeinrichtung 7 aufweisenden, in dem Gehäuse 11 drehbaren Rotor 12 umfaßt. Die Umlenkeinrichtung 7 bzw. die Reflexionsfläche 8 ist im Bereich eines Beleuchtungs- und Detektionsfensters 13 des Gehäuses 11 angeordnet.

Innerhalb des Rotors 12 ist eine den parallel zur Rotationsachse 14 verlaufenden Beleuchtungsstrahl 9 fokussierende Optik 15 angeordnet.

Bei dem in Fig. 1 dargestellten ersten Ausführungsbeispiel ist der Rotor 12 innerhalb des Gehäuses 11 frei drehbar und linear verschiebbar geführt bzw. gelagert. Sowohl zur Drehbewegung als auch zum linearen Vorschub des Rotors 12 ist ein gemeinsamer Rotorantrieb 16 vorgesehen, wobei dieser Antrieb über ein besonderes Übersetzungsgetriebe auf den Rotor 12 wirken kann.

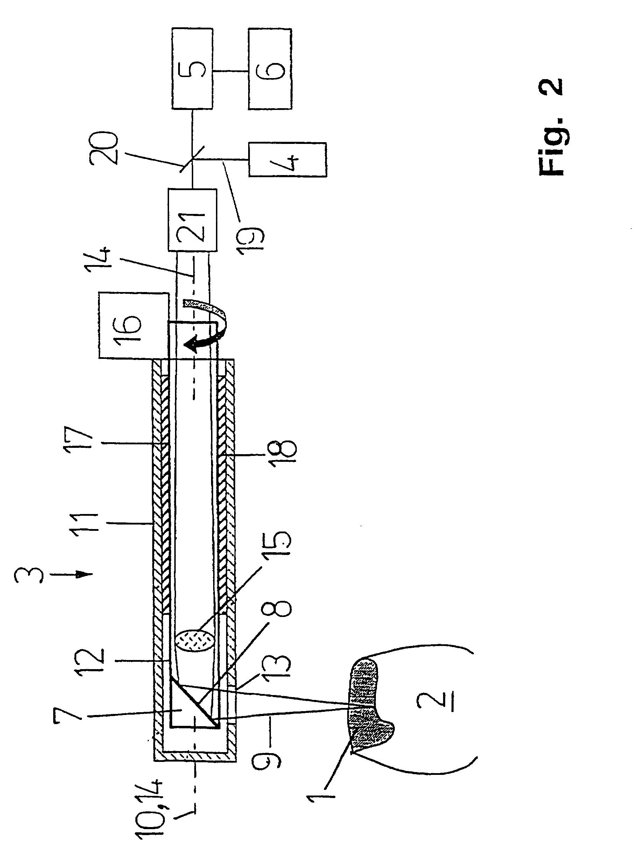

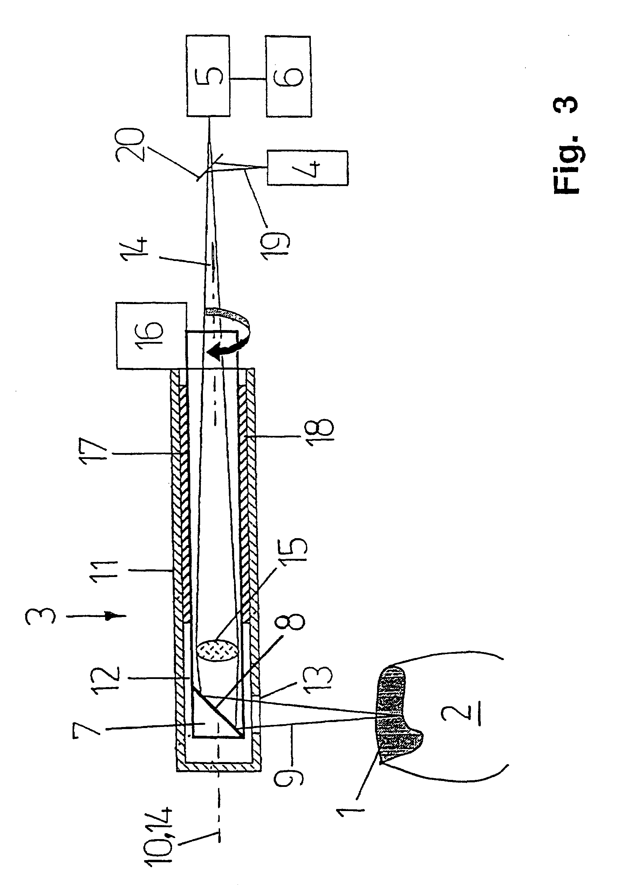

Bei den in den Figuren 2 und 3 dargestellten Ausführungsbeispielen ist der Rotor 12 mit einem Außengewinde 17 in einem Innengewinde 18 des Gehäuses 11 drehbar geführt. Dabei ist die Rotationsbewegung des Rotors 12 mit dessen linearen Vorschub zwangsgekoppelt. Die Gewinde 17, 18 sind als Feingewinde mit geringer Steigung ausgeführt, wobei auch dort lediglich ein Rotorantrieb 16 zur Drehung des Rotors und somit auch - zwangsweise - zum linearen Vorschub des Rotors 12 vorgesehen ist. Bei sämtlichen hier gezeigten Ausführungsbeispielen ist der Rotorantrieb 16 unmittelbar dem Gehäuse 11 zugeordnet und wirkt der Rotorantrieb 16 somit unmittelbar zwischen dem Gehäuse 11 und dem Rotor 12.

Bei den in den Figuren 1 und 2 dargestellten Ausführungsbeispielen ist die Lichtquelle 4 als Laserlichtquelle ausgeführt. Entsprechend ist der Lichtquelle 4 im Beleuchtungsstrahlengang 19 ein Strahlteiler 20 und eine Einrichtung 21 zur Fokuskontrolle bzw. Brennweitenänderung nachgeordnet.

Bei dem in Fig. 3 dargestellten Ausführungsbeispiel handelt es sich bei der Lichtquelle 4 um eine polyfokale Lichtquelle. Hier ist der Lichtquelle 4 im Beleuchtungsstrahlengang 19 lediglich ein Strahlteiler 20 nachgeordnet, wobei es sich bei dem Detektor 5 um einen polyfokalen Detektor handelt. Eine Einrichtung zur Fokuskontrolle bzw. Brennweitenänderung ist hier nicht erforderlich.

Bei dem in den Figuren gezeigten Ausführungsbeispielen erfolgt die Einspeisung des Lichts in die Sonde 3 und die Ableitung des Lichts aus der Sonde 3 über eine in den Figuren nicht gezeigte Lichtleitfaser, die als flexible Verbindung zwischen der Sonde 3 und der Lichtquelle 4 einerseits und den Auswerteeinheiten andererseits und somit zur Einspeisung des Lichts sowie zur Ableitung der durch Wechselwirkung des Lichts an der Oberfläche des zu scannenden Zahnes 2 entstehenden Signale dient.

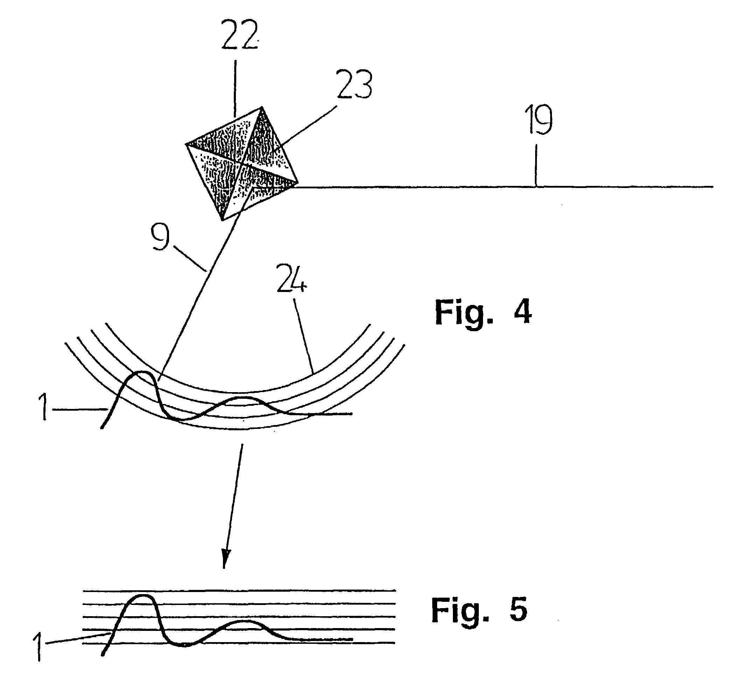

Die Figuren 4 und 5 zeigen eine schematische Darstellung des Scannvorganges bei rotierendem Lichtstrahl und linearem Vorschub, wobei dort als Umlenkeinrichtung 7 ein Polygon 22 vorgesehen ist. Der Beleuchtungsstrahl 9 wird an einer Facette 23 des Polygons 22 reflektiert und gelangt von dort auf das Oberflächenprofil 1 des Zahnes 2. Entlang der in Fig. 4 angedeuteten Zylindersegmente 24 wird das Oberflächenprofil 1 - Zylindersegment für Zylindersegment - gescannt, wobei sich unterschiedliche Brennebenen durch sequentielle oder simultane Abtastung sowohl hinsichtlich spekularer Reflexe als auch hinsichtlich schwachen Streulichts oder Fluoressenzlichts der jeweiligen Brennbene ergeben.

Gemäß der schematischen Darstellung in Fig. 5 werden die auf Zylindersegmente bezogenen Abtastwerte im Rahmen einer Geometriekorrektur auf eine "echte" Fokalebene transformiert, so daß sich aus den Abtastungen eine unverzerrte dreidimensionale Darstellung des Oberflächenprofils 1 bzw. des Zahnes 2 berechnen läßt.

Schließlich sei darauf hingewiesen, daß die voranstehend erörterten Ausführungsbeispiele lediglich zur Verdeutlichung der hier konkret beanspruchten Lehre dienen, diese jedoch nicht auf die Ausführungsbeispiele einschränken.

### Bezugszeichenliste

- 1: Oberflächenprofil, Oberfläche
- 2: Zahn
- 3: Sonde
- 4: Lichtquelle
- 5: Detektor
- 6: Prozessor
- 7: Umlenkeinrichtung
- 8: Reflexionsfläche
- 9: Beleuchtungsstrahl
- 10: Achse (zur linearen Bewegung des Rotors)
- 11: Gehäuse
- 12: Rotor
- 13: Beleuchtungs- und Detektionsfenster
- 14: Rotationsachse (des Rotors)
- 15: Optik
- 16: Rotorantrieb
- 17: Außengewinde (des Rotors)
- 18: Innengewinde (des Gehäuses)
- 19: Beleuchtungsstrahlengang
- 20: Strahlteiler
- 21: Einrichtung zur Fokuskontrolle
- 22: Polygon
- 23: Facette (des Polygons)
- 24: Zylindersegment (der Abtastung)

## Patentansprüche

1. Vorrichtung zur konfokalen Oberflächenvermessung in Körperhöhlen, insbesondere zur Vermessung des Oberflächenprofils (1) von Zähnen (2) in der Mundhöhle, mit einer in die Körperhöhle einführbaren Sonde (3), einer die Sonde (3) speisenden Lichtquelle (4), einem Lichtsignale aufnehmenden Detektor (5) und einem die detektierten Signale digitalisierenden und zu einer dreidimensionalen Darstellung weiterverarbeitenden Prozessor (6), **dadurch gekennzeichnet, daß** die Sonde (3) im Sinne eines Rotationsscanners mit einer Umlenkeinrichtung (7) ausgebildet ist, die den Beleuchtungsstrahl (9) in Richtung der zu vermessenden Oberfläche (1) lenkt, daß die Umlenkeinrichtung (7) zum Vorschub des rotierenden Beleuchtungsstrahls (9) in einer weiteren zu scannenden Achse (10) verschiebbar ist und daß vorzugsweise der Detektor (5) eine Einrichtung zur sequentiellen oder simultanen Abtastung mehrerer Brennebenen sowohl hinsichtlich spekularer Reflexe als auch hinsichtlich schwachen Streulichts oder Fluoreszenzlichts der jeweiligen Brennebene umfaßt.

2. Vorrichtung nach Anspruch 1, **dadurch gekennzeichnet, daß** die Umlenkeinrichtung (7) als Einfachspiegel ausgeführt ist.

3. Vorrichtung nach Anspruch 1, **dadurch gekennzeichnet, daß** die Umlenkeinrichtung (7) als Prisma ausgeführt ist.

4. Vorrichtung nach Anspruch 1, **dadurch gekennzeichnet, daß** die Umlenkeinrichtung (7) als Polygon (22) mit mehreren Facetten (23) zur Bildung mehrerer Strahlführungen ausgebildet ist.

5. Vorrichtung nach einem der Ansprüche 1 bis 4, **dadurch gekennzeichnet, daß** die Sonde (3) ein Gehäuse (11) mit einem die Umlenkeinrichtung (7) aufweisenden, in dem Gehäuse (11) drehbaren Rotor (12) umfaßt und daß die Umlenkeinrichtung (7) im Bereich eines Beleuchtungs- und Detektionsfensters (13) des Gehäuses (11) angeordnet ist.

6. Vorrichtung nach einem der Ansprüche 1 bis 4, **dadurch gekennzeichnet, daß** innerhalb des Rotors (12) eine den parallel zur Rotationsachse (14) verlaufenden Beleuchtungsstrahl (9) fokussierende Optik (15) angeordnet ist.

7. Vorrichtung nach Anspruch 5 oder 6, **dadurch gekennzeichnet, daß** der Rotor (12) innerhalb des Gehäuses (11) frei drehbar und linear verschiebbar geführt bzw. gelagert ist und daß ein Rotorantrieb (16) zur Drehbewegung und zum linearen Vorschub des Rotors (12) vorgesehen ist.

8. Vorrichtung nach Anspruch 7, **dadurch gekennzeichnet, daß** für die Drehbewegung und für den linearen Vorschub des Rotors (12) zwei unabhängige Antriebe vorgesehen sind.

9. Vorrichtung nach Anspruch 5 oder 6, **dadurch gekennzeichnet, daß** der Rotor (12) mit einem Außengewinde (17) in einem Innengewinde (18) des Gehäuses (11) drehbar geführt ist, so daß die Rotationsbewegung des Rotors (12) mit dessen Vorschub zwangsgekoppelt ist.

10. Vorrichtung nach Anspruch 9, **dadurch gekennzeichnet, daß** die Gewinde (17, 18) als Feingewinde mit geringer Steigung ausgeführt sind.

11. Vorrichtung nach Anspruch 9, **dadurch gekennzeichnet, daß** die Gewinde (18, 18) als Differentialgewinde mit sehr kleinem Vorschub, insbesondere im Bereich von vorzugsweise 50 Mikrometer pro Scanzeile, ausgeführt sind.

12. Vorrichtung nach einem der Ansprüche 7 bis 11, **dadurch gekennzeichnet, daß** der Rotorantrieb (16) unmittelbar dem Gehäuse (11) zugeordnet ist.

13. Vorrichtung nach einem der Ansprüche 1 bis 12, **dadurch gekennzeichnet, daß** es sich bei der Lichtquelle (4) um eine Laserlichtquelle, insbesondere um einen Diodenlaser, handelt.

14. Vorrichtung nach einem der Ansprüche 1 bis 12, **dadurch gekennzeichnet, daß** der Lichtquelle (4) im Beleuchtungsstrahlengang (19) ein Strahlteiler (20) und eine Einrichtung zur Fokuskontrolle bzw. zur Brennweitenänderung nachgeordnet ist.

15. Vorrichtung nach einem der Ansprüche 1 bis 12, **dadurch gekennzeichnet, daß** es sich bei der Lichtquelle (4) um eine polyfokale Lichtquelle handelt.

16. Vorrichtung nach Anspruch 15, **dadurch gekennzeichnet, daß** der Lichtquelle im Beleuchtungsstrahlengang (19) ein Strahlteiler (20) nachgeordnet ist und daß es sich bei dem Detektor (5) um einen polyfokalen Detektor handelt.

17. Vorrichtung nach einem der Ansprüche 1 bis 16, **dadurch gekennzeichnet, daß** die Einspeisung des Lichts in die Sonde (3) und die Ableitung des Signals aus der Sonde (3) über eine Lichtleitfaser erfolgt.

18. Vorrichtung nach einem der Ansprüche 1 bis 17, **dadurch gekennzeichnet, daß** die Lichtquelle (4) und/oder der Strahlteiler (20) und/oder die Fokuskontrolle und/oder der Detektor (5) und/oder der Prozessor (6) innerhalb des Gehäuses (11) angeordnet sind.

19. Vorrichtung nach einem der Ansprüche 1 bis 18, **dadurch gekennzeichnet, daß** der Prozessor (6) zur Steuerung, Transformation bzw. Geometriekorrektur und Digitalisierung der Signale, zur Berechnung des dreidimensionalen Oberflächenprofils (1) sowie zur Speicherung der Daten dient.

## Claims

1. Apparatus for confocal surface measurement in body cavities, in particular for measuring the surface profile (1) of teeth (2) in the oral cavity, having a probe (3) which can be inserted into the body cavity, a light source (4) feeding the probe (3), a detector (5) which picks up light signals, and a processor (6) which digitizes the detected signals and processes them further to form a three-dimensional display, **characterized in that** the probe (3) is designed as a rotary scanner with a deflecting device (7) which directs the illuminating beam (9) in the direction of the surface (1) to be measured, **in that** the deflecting device (7) can be displaced in order to advance the rotating illuminating beam (9) along a further axis (10) to be scanned, and **in that** preferably the detector (5) comprises a device for sequential or simultaneous scanning of a plurality of focal planes both with regard to specular reflections and with regard to weak scattered light or fluorescent light of the respective focal plane.

2. Apparatus according to Claim 1, **characterized in that** the deflecting device (7) is designed as a single mirror.

3. Apparatus according to Claim 1, **characterized in that** the deflecting device (7) is designed as a prism.

4. Apparatus according to Claim 1, **characterized in that** the deflecting device (7) is designed as a polygon (22) with a plurality of facets (23) for forming a plurality of beam guides.

5. Apparatus according to one of Claims 1 to 4, **characterized in that** the probe (3) comprises a housing (11) with a rotor (12) which has the deflecting device (7) and can be rotated in the housing (11), and **in that** the deflecting device (7) is arranged in the region of an illumination and detection window (13) of the housing (11).

6. Apparatus according to one of Claims 1 to 4, **characterized in that** an optical system (15) which focuses the illuminating beam (9) running parallel to the axis of rotation (14) is arranged inside the rotor (12).

7. Apparatus according to Claim 5 or 6, **characterized in that** the rotor (12) is guided or mounted inside the housing (11) in a freely rotatable and linearly displaceable fashion, and **in that** a rotor drive (16) is provided for the rotary movement and for the linear advance of the rotor (12).

8. Apparatus according to Claim 7, **characterized in that** two independent drives are provided for the rotary movement and for the linear advance of the rotor (12).

9. Apparatus according to Claim 5 or 6, **characterized in that** the rotor (12) is relatively guided with the aid of an external thread (17) in an internal thread (18) of the housing (11) such that the rotary movement of the rotor (12) is positively coupled to its advance.

10. Apparatus according to Claim 9, **characterized in that** the threads (17, 18) are designed as fine threads with a low pitch.

11. Apparatus according to Claim 9, **characterized in that** the threads (17, 18) are designed as differential threads with very small advance, in particular in the region of preferably 50 micrometres per scanning line.

12. Apparatus according to one of Claims 7 to 11, **characterized in that** the rotor drive (16) is arranged directly at the housing (11).

13. Apparatus according to one of Claims 1 to 12, **characterized in that** the light source (4) is a laser light source, in particular a diode laser.

14. Apparatus according to one of Claims 1 to 12, **characterized in that** a beam splitter (20) and a device for focus control or for changing the focal length are arranged downstream of the light source (4) in the illuminating beam path (19).

15. Apparatus according to one of Claims 1 to 12, **characterized in that** the light source (4) is a polyfocal light source.

16. Apparatus according to Claim 15, **characterized in that** a beam splitter (20) is arranged downstream of the light source in the illuminating beam path (19), and **in that** the detector (5) is a polyfocal detector.

17. Apparatus according to one of Claims 1 to 16, **characterized in that** the light is fed into the probe (3) and the signal is led off from the probe (3) via an optical fibre.

18. Apparatus according to one of Claims 1 to 17, **characterized in that** the light source (4) and/or the beam splitter (20) and/or the focus control and/or the detector (5) and/or the processor (6) are arranged inside the housing (11).

19. Apparatus according to one of Claims 1 to 18, **characterized in that** the processor (6) is used to control, transform or correct the geometry and digitize the signals, to calculate the three-dimensional surface profile (1) and to store the data.

## Revendications

1. Dispositif de mesure confocale de surfaces dans les cavités corporelles, en particulier de mesure du profil de surface (1) de dents (2) dans la cavité buccale, à l'aide d'une sonde (3) pouvant être introduite dans la cavité corporelle, une source lumineuse (4) alimentant la sonde (3), un détecteur (5) captant les signaux lumineux et un processeur (6) qui numérise les signaux détectés et les traite de manière à obtenir une représentation en trois dimensions, **caractérisé en ce que** la sonde (3) est réalisée à la manière d'un scanner rotatif avec un dispositif de modification de direction (7), lequel dirige le faisceau d'éclairage (9) en direction de la surface (1) à mesurer, que le dispositif de modification de direction (7) pour l'avance du faisceau d'éclairage (9) rotatif peut être déplacé selon un axe supplémentaire (10) à scanner et que le détecteur (5) comprend de préférence un dispositif pour le balayage séquentiel ou simultané de plusieurs plans focaux pour ce qui est des réflexes spéculaires et de la faible lumière diffusée ou lumière fluorescente des plans focaux respectifs.

2. Dispositif selon la revendication 1, **caractérisé en ce que** le dispositif de modification de direction (7) est réalisé sous forme d'un simple miroir.

3. Dispositif selon la revendication 1, **caractérisé en ce que** le dispositif de modification de direction (7) est réalisé sous forme d'un prisme.

4. Dispositif selon la revendication 1, **caractérisé en ce que** le dispositif de modification de direction (7) est réalisé sous forme d'un polygone (22) à plusieurs facettes (23) pour former plusieurs directions de rayons.

5. Dispositif selon l'une quelconque des revendications 1 à 4, **caractérisé en ce que** la sonde (3) comprend un boîtier (11) avec un rotor orientable (12) à l'intérieur du boîtier (11) qui comprend le dispositif de modification de direction (7), et que le dispositif de modification de direction (7) est aménagé dans la zone d'une fenêtre d'éclairage et de détection (13) du boîtier (11).

6. Dispositif selon l'une quelconque des revendications 1 à 4, **caractérisé en ce qu'**à l'intérieur du rotor (12), est aménagée une optique focalisante (15) du faisceau d'éclairage (9) s'étendant parallèlement à l'axe de rotation (14).

7. Dispositif selon la revendication 5 ou 6, **caractérisé en ce que** le rotor (12) à l'intérieur du boîtier (11) est entraîné ou logé de sorte à pivoter librement et être déplacé linéairement et qu'un entraînement de rotor (16) est prévu pour obtenir un mouvement rotatif et un déplacement en avant linéaire du rotor (12).

8. Dispositif selon la revendication 7, **caractérisé en ce que**, pour le mouvement rotatif et pour le déplacement en avant linéaire du rotor (12), deux entraînements indépendants sont prévus.

9. Dispositif selon la revendication 5 ou 6, **caractérisé en ce que** le rotor (12) est guidé de manière à pouvoir tourner avec un filetage extérieur (17) dans un filetage intérieur (18) du boîtier (11), moyennant quoi le mouvement de rotation du rotor (12) est couplé de manière forcée avec son déplacement en avant.

10. Dispositif selon la revendication 9, **caractérisé en ce que** les filetages (17, 18) se présentent sous forme de filetages fins avec un faible pas.

11. Dispositif selon la revendication 9, **caractérisé en ce que** les filetages (17, 18) se présentent sous forme de filetages différentiels avec un très faible déplacement vers l'avant, en particulier dans la plage comportant de préférence 50 micromètres par ligne de balayage.

12. Dispositif selon l'une quelconque des revendications 7 à 11, **caractérisé en ce que** l'entraînement du rotor (16) est disposé près du boîtier (11).

13. Dispositif selon l'une quelconque des revendications 1 à 12, **caractérisé en ce que** la source lumineuse (4) est une source lumineuse laser, en particulier un laser à diode.

14. Dispositif selon l'une quelconque des revendications 1 à 12, **caractérisé en ce que** sont disposés dans le faisceau d'éclairage (19) un diviseur de rayonnement (20) et un dispositif pour le contrôle de focalisation ou la modification de la distance focale après la source lumineuse (4).

15. Dispositif selon l'une quelconque des revendications 1 à 12, **caractérisé en ce que** la source lumineuse (4) est une source lumineuse polyfocale.

16. Dispositif selon la revendication 15, **caractérisé en ce qu'**après la source lumineuse est disposé, dans le faisceau d'éclairage (19), un diviseur de rayonnement (20) et que le détecteur (5) est un détecteur polyfocal.

17. Dispositif selon l'une quelconque des revendications 1 à 16, **caractérisé en ce que** l'alimentation en lumière de la sonde (3) et la dérivation du signal de la sonde (3) s'effectuent via une fibre optique.

18. Dispositif selon l'une quelconque des revendications 1 à 17, **caractérisé en ce que** la source lumineuse (4) et/ou le diviseur de rayonnement (20) et/ou le contrôle de focalisation et/ou le détecteur (5) et/ou le processeur (6) sont aménagés à l'intérieur du boîtier (11).

19. Dispositif selon l'une quelconque des revendications 1 à 18, **caractérisé en ce que** le processeur (6) sert au pilotage, à la transformation ou la correction géométrique et la numérisation des signaux, au calcul du profil de la surface (1) en trois dimensions ainsi qu'à l'enregistrement des données.
